# NEW EUROPEAN PATENT SPECIFICATION

(11) **EP 0 774 987 B2**
(45) Date of publication and mention of the opposition decision: **11.01.2006**
(45) Mention of the grant of the patent: 02.06.1999
(21) Application number: 94918157.2
(22) Date of filing: 27.05.1994
(51) Int. Cl.: A61M 25/10, A61M 29/02

(54) **SELECTIVE ARRANGEMENT OF LUBRICOUS COATINGS ON BALLOON CATHETERS**
SELEKTIVE ANORDNUNG VON GLEITBESCHICHTUNGEN AUF BALLONKATHETERN
DISPOSITION SELECTIVE DE COUCHES LUBRIFIANTES SUR DES CATHETERS A BALLONNET

(30) Priority: 02.06.1993 US 70601
(43) Date of publication of application: 28.05.1997
(73) Proprietor: Boston Scientific Scimed, Inc., Maple Grove, MN 55311 (US)
(72) Inventor: SLAIKEU, Paul, C., Vadnais Heights, MN 55127 (US); KLEE, James, Maple Grove, MN 55369 (US); DUGGAN, Roger, Memphis, TN 38104 (US)
(74) Representative: Maiwald, Walter
(86) International application number: PCT/US1994/006024
(87) International publication number: WO 1994/027665

(56) References cited:
- EP-A- 0 380 102
- EP-A- 0 592 320
- WO-A-91/08790
- WO-A-94/07561
- JP-A- 4 271 436
- US-A- 2 927 584
- US-A- 5 041 100
- US-A- 5 047 045
- US-A- 5 100 381
- US-A- 5 129 890
- US-A- 5 135 474
- US-A- 5 135 516
- US-A- 5 156 595
- US-A- 5 176 698
- US-A- 5 183 464
- US-A- 5 209 730

## Description

### Background of the Invention

This invention relates to balloon catheters, sometimes referred to as dilatation catheters, for use in angioplasty. Angioplasty has become recognized as an efficient and effective method of opening stenoses in the vascular system. In the most widely used form of angioplasty, a balloon catheter is guided through the vascular system until the balloon, which is carried at the distal end of a catheter shaft, is positioned across the stenosis or lesion, i.e., vessel obstruction. The balloon is then inflated to apply pressure to the obstruction which is essentially remolded by pressing it against the inner wall of the vessel whereby the vessel is opened for improved flow.

Balloon catheters are of various types. One type is fed over a guide wire (i.e., "over-the-wire" catheters) and another type serves as its own guide wire (i.e., "fixed-wire" catheters). Variations of these two basic types also have been developed such as the so called "rapid exchange" type, "innerless" catheters, and others. As used herein, the term "balloon catheter" is meant to include all of the various types of angioplasty catheters which carry a balloon for performing angioplasty. Balloon catheters may also be of a wide variety of inner structure, such as different lumen design, of which there are at least three basic types: triple lumen, dual lumen and coaxial lumen. All varieties of internal structure and design variation are meant to be included by use of the term "balloon catheter" herein.

When used in percutaneous transluminal coronary angioplasty (PTCA), the balloon catheter is typically advanced through a guide catheter to a preselected vessel location such as the aorta. Using fluoroscopy, the surgeon manipulates the catheter until the balloon is located across the stenosis or obstruction. As already pointed out, this may involve the use of a guide wire over which the catheter is moved or alternatively the catheter may act as its own guide wire, depending on the particular design. The manipulation of the balloon catheter through the guide catheter and through the vessels to the obstruction requires the balloon catheter to have a number of different features.

One such feature is the use of a lubricous coating over the exterior surfaces of the catheter and balloon to facilitate movement of the catheter through the sometimes tortuous paths within the vascular system to the preselected vessel location for performing the angioplasty. A wide variety of such lubricous coatings have become commonplace for use with respect to catheters and other devices which are insertable into the body in connection with surgical procedures and the like. All such coatings are intended to be included herein with respect to the use of the term "lubricous coating". Examples of such coatings indude silicone and most preferably hydrophilic coatings involving hydrogel polymers or the like, such as polymer networks of a vinyl polymer and an uncrosslinked hydrogel, for example. Polyethylene oxide (PEO) is a preferred hydrogel. A preferred vinyl polymer is neopentyl glycol diacrylate (NPG).

EP-A-0 380 102 upon which the preamble of claim 1 is based discloses a balloon catheter of the type including a shaft and a balloon associated therewith, the balloon having a proximal end portion, a distal end portion and a central portion, and including a lubricous means with said balloon catheter, said lubricous being bonded to the balloon catheter. The balloon catheter of the present application differs from the prior art in that, the lubricous means is constructed and arranged to provide relatively more lubricity with respect to a major portion of the shaft than with respect to at least a portion of the balloon. In contrary to EP-A-0 380 102 the present invention provides a means of anchoring or positioning the balloon in a fixed location so as to avoid unexpected movement thereof upon balloon expansion.

These coatings have even been known to include certain agents such as drugs which may be permanently entrapped in the coating or leachable therefrom into the body. For example, heparin has been used in such a fashion. Heparin is well known as an agent which is often used to inhibit clot formation in the blood. Again, the term "lubricous coating" is meant to include all such variations.

The term "watermelon seeding" refers to the commonly experienced phenomenon which occurs when one squeezes a watermelon seed between the forefinger and thumb, the result of which usually results in the flight of the watermelon seed. Thus, this terminology aptly describes the potential problem which might occur in a situation in which a lubricated balloon has been positioned across an obstruction and inflated. As a result of the increased pressure exerted by the expanded balloon against the obstruction, an unexpected movement of the balloon in one direction or the other in the vessel might occur as the balloon slips along the obstruction in an attempt to relieve the increased pressure. This unexpected movement might be regarded negatively by a surgeon. Thus, through high lubricity is desirable for general movement, it is also desirable to provide a means of anchoring or positioning the balloon in fixed location so as to avoid unexpected movement thereof upon balloon expansion.

### Summary of the Invention

This is accomplished by the present invention through the selective arrangement of lubricous coatings on the balloon catheter in which at least a substantial portion of the balloon body is uncoated or less slippery while a relatively more lubricous coating is placed on at least a substantial portion of the catheter shaft extending in a proximal direction from the balloon toward the proximal end of the catheter shaft.

Generally then, the invention contemplates an improvement in the arrangement of lubricous coating(s) on the catheter to avoid "watermelon seeding" and to better anchor the balloon in position for performing angioplasty. This is accomplished by what may be termed herein as "differential coating" or "selective lubricating". By this is meant that the lubricous properties of the catheter are selectively designed or constructed and arranged in a predetermined manner such that the catheter shaft i.e. substantially all of the catheter generally exhibits more lubricity than the balloon generally. In short, the catheter is more slippery than the balloon, relatively speaking. It can be seen that an important feature of the invention lies in the uncoated or less slippery balloon or portion thereof relative to the rest of the catheter.

In a preferred embodiment, the catheter shaft and balloon are both, lubricously coated, but the coating on the balloon is less lubricous or less slippery than the one on the catheter shaft. In such an instance, two different coatings may be used, such as a PEO composition as aforementioned on the catheter shaft and so forth while a silicone coating is placed on the balloon per se. Such an arrangement represents a preferred embodiment of the invention.

In another variation, different compositions of PEO may be used on the shaft and balloon. In the PEO compositions aforementioned, comprised of PEO and NPG in isopropyl alcohol and water, variations in the amount of PEO content affect the final lubricity of the composition; the higher the percentage PEO, the higher the lubricity. Therefore, one may utilize a relatively higher percentage PEO composition on a catheter shaft and a relatively low percentage composition on the balloon to achieve the ends of this invention also i.e., "differential" or "selective" lubricity over a catheter. Of course, other compositions may also be utilized in this way.

Another preferred embodiment comprises a coated catheter shaft, coated balloon cones and a coating on at least the distal waist of the balloon extending to the distal catheter tip, the balance of the balloon body having no coating or one of less lubricity.

Additionally and even more preferably both the proximal and distal balloon cones are so coated similarly to the catheter shaft or at least the distal cone. In accordance with the invention, the balance of the balloon body is uncoated or coated with a relatively less lubricous coating.

### Brief Description of the Drawings

A detailed description of the invention is hereafter described with specific reference being made to the drawings in which:
FIG. 1 is a diagrammatic showing of a balloon catheter in which the balloon is positioned across an obstruction in a vessel prior to inflation.
FIG. 2 is a diagrammatic showing of the positioned balloon catheter of Figure 1 with the balloon inflated against the obstruction.
FIG. 3 is a schematic showing of a typical balloon catheter.
FIG. 4 is a schematic diagram showing the basic anatomy of a catheter balloon per se coated according to one preferred embodiment of the invention.

### Detailed Description of the Invention

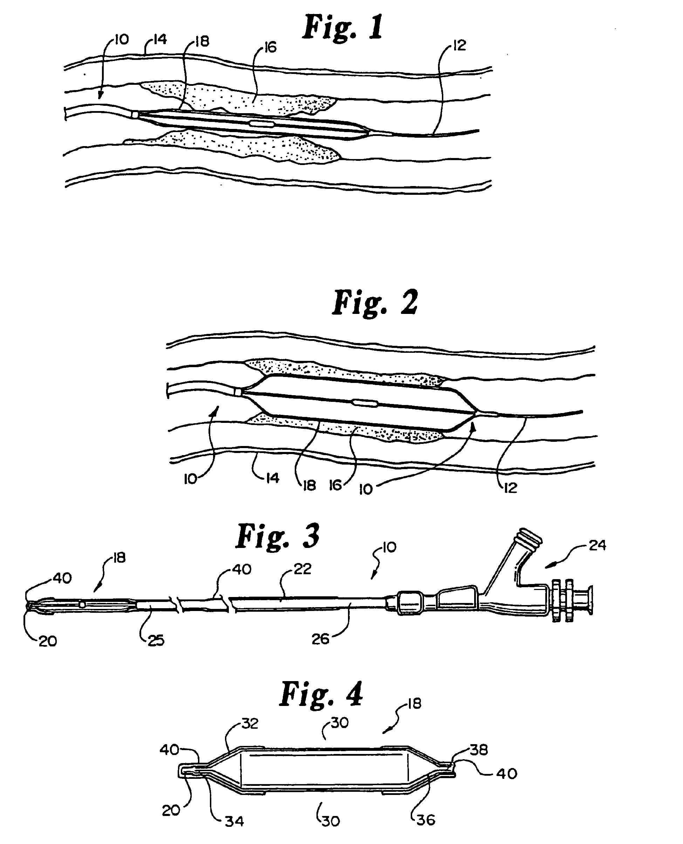

Referring now to Figures 1 and 2, reference to the aforementioned "watermelon seeding" effect will be more readily understood. Figures 1 and 2 show a balloon catheter, generally indicated at 10, of the over-the-wire type having a guide wire 12 over which the catheter has been moved within a vessel 14 to the location of an obstruction 16. As can be seen in the Figures, balloon 18 is positioned across obstruction 16 while uninflated (shown in Figure 1). Upon inflation (shown in Figure 2), as the balloon expands and exerts pressure against obstruction 16 it is possible for the "watermelon seeding" effect to occur if care is not taken to hold the catheter in a fixed position. As shown in Figure 2, if the catheter is held in a fixed position, the expanding balloon presses against obstruction 16 molding it against the inner walls of vessel 14 to open the vessel. As already indicated with respect to the present invention, the surface of balloon 18 shown contacting obstruction 16 is best not coated or coated with a less lubricous coating than the catheter proper in order to provide an "anchoring" effect when it engages the obstruction. This is more fully described hereinbelow with reference to Figures 3 and 4.

Figure 3 shows a typical balloon catheter which may incorporate any of the various aforementioned design variations for catheters. For purposes of understanding the present invention it is only important to note that catheter 10 includes a balloon generally indicated at 18, a distal tip 20, a shaft 22 and a manifold portion generally indicated at 24. Shaft 22 is comprised of a proximal end portion 26 and a distal end portion 25 where it joins balloon 18.

As can be seen in Figure 4, balloon 18 indudes a body portion 30, a proximal cone portion 36, a proximal waist portion 38 and a distal cone portion 32 along with a distal waist portion 34.

In accordance with one embodiment of the selective arrangement of the lubricous coatings on the catheter as contemplated herein, such a coating is shown at 40 extending over proximal cone 36 (optional), in a proximal direction over shaft 22 toward the proximal end portion 26 thereof to thereby cover a substantial portion of shaft 22. Coating 40 is also optionally included on the distal cone 32, distal waist portion 34 and the distal tip of the catheter 20 as can be seen best in Figures 3 and 4. The balance 30 of the balloon is either uncoated or coated with a less lubricous composition.

As is known in the art with respect to balloon catheters and lubricous coatings, the coating will be relatively thin and preferably bonded to the catheter body surfaces, although be not necessarily. Silicone is an example of an unbonded lubricant. The PEO based coating cited earlier is an example of a bonded one. In the Figures, the relative thickness of coating is greatly exaggerated for clarity. However, in accordance with standard practices in the art, such coatings may nominally be of a thickness on the order of 20-50 µm or less and will be applied in a variety of ways depending on the type of coating involved and the particular selective arrangement of the coating desired. For example, in the ease of various polymeric hydrophilic coatings it has been found convenient to utilize an elastic mask to block the body portion of the balloon from being coated with the slippery hydrophilic coating. The mask, in a preferred form, is a heat shrink polyolefin sized to provide a slight interference fit around the balloon to keep the mask in place during processing. In one preferred form, the uncoated length of the area masked has been about 15,9 mm (5/8 of an inch) centered on the body portion of the balloon and extending around its peripheral surface. Such an arrangement is indicated in Figure 4. Of course, the entire body per se of the balloon may be left uncoated as well or coated with a relatively less lubricous coating than the balance of the catheter proper.

Upon completion of the polymer coating procedure, the mask is then removed to expose the uncoated balloon body portion. In such an arrangement, the shaft and balloon cones and waists, being unmasked during the coating procedure, are coated with the same slippery hydrophilic coating as is placed on the rest of the catheter. As is already known, such coatings are typically applied to the catheter surfaces in the form of a solution which is allowed to dry and is subsequently cured usually by heat or Ultraviolet light for a short period of time.

Other means for achieving the selective placement of coating(s) on the catheter may include the use of a release agent such as an oil which may be spread over the area which it is desired will remain uncoated. After curing of the coating, this area is then exposed by simply peeling the coating off the area carrying the oil. Also, if desired, one may rinse or wipe a portion of the coating off the balloon before the coating is cured. Another alternative is to modify the coating in the area of the balloon which is to remain uncoated such as using an ultraviolet blocker.

A final selective coating arrangement according to the invention may also be made by first coating with a less slippery, compatible coating over the balloon and possibly more of the catheter, then masking, applying highly slippery coatings as desired and proceeding as normal or vice versa.

This invention is equally applicable to balloons of the compliant type and to those of the non-compliant type. A wide variety for the materials of the balloons is well known, some examples of which, to name a few, include ethylene vinylacetate copolymer polyethylene terephthalate, polyethylene, polyolefin copolymer and high density polyethylene.

Masking materials are most conveniently heat shrink polyethylene on mandrels to sizes appropriate to the particular balloon and are placed thereon. The coating may comprise a mix of a higher molecular weight soluble polymer such as PEO and a UV curable diacrylate in isopropyl alcohol and water containing a trace of phatoinitiator. The coating solution is wiped onto selected areas of the catheter device which is then passed to a UV chamber, purged of oxygen, exposed to UV and then removed. The mask is removed, the area is cleaned ultrasonically with a water bath to remove any drips. The uncoated portion of the balloon is left as is or a less lubricous coating is applied to it such as silicone or a polymer coating with a lesser percentage of hydrogel content Other methods of application will be known to those familiar with the art.

While this invention may be embodied in many different forms, there are shown in the drawings and described in detail herein specific preferred embodiments of the invention. The present disclosure is an exemplification of the principles of the invention and is not intended to limit the invention to the particular embodiments illustrated.

This completes the description of the preferred and alternate embodiments of the invention. Those skilled in the art may recognize other equivalents to the specific embodiment described herein which equivalents are intended to be encompassed by the claims attached hereto.

## Claims

1. A balloon catheter (10) including a shaft (22) and a balloon (18) associated therewith, the balloon (18) having a proximal end portion, a distal end portion, a central portion (30), and a first lubricous means with said balloon catheter **characterized in that**, said first lubricous means being bonded to the balloon catheter (10) and being constructed and arranged to provide more lubricity with respect to a major portion of the shaft (22) than with respect to at least a portion of the balloon (18).

2. The catheter according to claim 1 wherein the first lubricous means is constructed and arranged to provide a lubricous coating on the shaft portion (18) and at least a portion of the balloon (22) thereof is uncoated.

3. The catheter according to claim 2 wherein the central portion of the balloon (30) is uncoated.

4. The catheter according to claim 3 further including a lubricous coating on the distal end portion of the balloon.

5. The catheter according to claim 4 wherein the proximal end portion of the balloon also includes a lubricous coating.

6. The catheter according to claim 1 including a second lubricous means which is relatively less lubricous than the first lubricous means.

7. The catheter according to claim 1 wherein the first lubricous means is constructed and arranged to provide a lubricous coating on the shaft (22), said catheter further comprising the second lubricous means constructed and arranged to provide a lubricous coating on at least a portion of the balloon, said lubricous coating on the balloon being less lubricous than the lubricous coating on the shaft.

8. The catheter according to claim 7 wherein the more as well as the highly lubricous coating comprises a polyethylene oxide composition.

9. The catheter according to claim 7 wherein the less lubricous coating comprises silicone.

10. The catheter according to claim 7 wherein the less lubricous coating is only a central portion of the balloon (30).

11. The catheter according to any of the preceding claims with a balloon (18) having opposite ends, a cone (32, 36) and a waste (34, 38) at each end, a distal tip portion (20), surface areas and lubricous coatings associated with the surface areas thereof wherein more lubricous and relatively less lubricous coatings associated with the surface areas thereof are predetermined arranged whereby the shaft (22) is provided with a relatively highly lubricous coating bonded to a substantial length thereof extending from the balloon (18) toward the proximal end of the shaft (26), both cones of the balloon (32, 36) are provided with a similar relatively highly lubricous coating as is the distal tip portion (20), while at least a substantial portion of the balloon body is coated with a lubricous coating that is relatively less lubricous than said highly lubricous coating.

12. The catheter according to claim 11 wherein the entire balloon body is coated with the less lubricous coating.

13. The catheter according to claim 11 wherein the central portion of the balloon body (30) is uncoated.

14. The catheter according to claim 11 wherein only the central portion of the balloon body (30) is coated with the less lubricous coating.

15. The catheter according to claim 11 wherein the proximal end portion of the shaft (26) is uncoated.

16. The catheter according to claim 11 wherein at least the distal waist of the balloon (34) is also coated with the highly lubricous coating.

17. The catheter according to claim 16 including the same highly lubricous coating on the proximal waist of the balloon (38).

18. The catheter according to claim 11 wherein only the distal cone (32) is coated with the highly lubricous coating.

19. The catheter according to claim 18 wherein the central portion of the balloon (30) is uncoated.

20. The catheter according to claim 18 wherein the central portion of the balloon (30) is coated with the less lubricous coating.

## Patentansprüche

1. Ein Ballonkatheter (10), der einen Schaft (22) und einen damit verbundenen Ballon (18), sowie ein erstes Schmiermittel zusammen mit genanntem Ballonkatheter umfasst, wobei der Ballon (18) ein proximales Ende, ein distales Ende und ein Mittelteil (30) aufweist, **dadurch gekennzeichnet, dass** das genannte erste Schmiermittel auf dem Ballonkatheter (10) angebracht und so zusammengesetzt und angeordnet ist, dass es einem größeren Teil des Schaftes (22) gegenüber wenigstens einem Teil des Ballons (18) eine größere Schlüpfrigkeit verschafft.

2. Der Katheter nach Anspruch 1, wobei das erste Schmiermittel so zusammengesetzt und angeordnet ist, dass eine Schmierschicht auf dem Schaftteil (18) gebildet wird und wenigstens ein Teil des Ballons (22) unbeschichtet ist.

3. Der Katheter nach Anspruch 2, wobei das Mittelteil des Ballons (30) unbeschichtet ist.

4. Der Katheter nach Anspruch 3, wobei weiterhin eine Schmierschicht auf dem distalen Ende des Ballons aufgebracht ist.

5. Der Katheter nach Anspruch 4, wobei das proximale Ende des Ballons auch eine Schmierschicht aufweist.

6. Der Katheter nach Anspruch 1, welcher ein zweites Schmiermittel umfasst, das weniger schmiert, als das erste Schmiermittel.

7. Der Katheter nach Anspruch 1, wobei das erste Schmiermittel so zusammengesetzt und angeordnet ist, dass eine Schmierschicht auf dem Schaft (22) gebildet wird und der genannte Katheter weiterhin das zweite Schmiermittel aufweist, das so zusammengesetzt und angeordnet ist, dass es eine Schmierschicht auf wenigstens einem Teil des Ballons bildet, wobei die genannte Schmierschicht auf dem Ballon weniger schmierend ist als die Schmierschicht auf dem Schaft.

8. Der Katheter nach Anspruch 7, wobei die hochschmierende Schicht eine Zusammensetzung aus Polyethylenoxid aufweist.

9. Der Katheter nach Anspruch 7, wobei die weniger schmierende Schmierschicht Silicon enthält.

10. Der Katheter nach Anspruch 7, wobei die weniger schmierende Schmierschicht nur auf einem Mittelteil des Ballons (30) befindlich ist.

11. Der Katheter nach einem der vorhergehenden Ansprüche, mit einem Ballon (18), der gegenüberliegende Enden, an jedem Ende einen Konus (32, 36) und eine Taille (34, 38), ein distales Spitzenstück (20), Oberflächen und Schmierschichten, die auf den Oberflächen aufgebracht sind, aufweist, und bei den auf die Oberflächen aufgebrachte stärker bzw. weniger schmierenden Schmierschichten in vorbestimmter Weise angeordnet sind, wobei auf den Schaft (22) in einem wesentlichen Längenbereich, der sich von dem Ballon (18) in Richtung auf das proximale Ende des Schaftes (26) erstreckt, eine in relativ hohem Maße schmierende Schicht aufgebracht ist, und beide Konus des Ballons (32, 36), wie auch das distale Spitzenstück (20) mit einer hierzu ähnlichen, in relativ hohem Maße schmierenden Schicht, ausgestattet sind, während wenigstens ein wesentlicher Teil des Ballonrumpfes mit einer Schmierschicht versehen ist, die weniger schmierend ist, als die genannte, in hohem Maße schmierende Schicht.

12. Der Katheter nach Anspruch 11, wobei der gesamte Ballonrumpf mit der weniger schmierenden Schicht beschichtet ist.

13. Der Katheter nach Anspruch 11, wobei das Mittelstück des Ballonrumpfes (30) unbeschichtet ist.

14. Der Katheter nach Anspruch 11, wobei nur das Mittelstück des Ballonrumpfes (30) mit der weniger schmierenden Schmierschicht beschichtet ist.

15. Der Katheter nach Anspruch 11, wobei das proximale Endstück des Schaftes (26) unbeschichtet ist.

16. Der Katheter nach Anspruch 11, wobei wenigstens die distale Engstelle des Ballons (34) ebenso mit der in hohem Maße schmierenden Schicht beschichtet ist.

17. Der Katheter nach Anspruch 16, wobei die gleiche, in hohem Maße schmierende Schicht auf der proximalen Taille des Ballons (38) vorgesehen ist.

18. Der Katheter nach Anspruch 11, wobei nur der distale Konus (32) mit der in relativ hohem Maße schmierenden Schicht beschichtet ist.

19. Der Katheter nach Anspruch 18, wobei das Mittelstück des Ballons (30) nicht beschichtet ist.

20. Der Katheter nach Anspruch 18, wobei das Mittelstück des Ballons (30) mit der weniger schmierenden Schmierschicht beschichtet ist.

## Revendications

1. Cathéter à ballonnet (10) comportant un tube (22) et un ballonnet (18) associé à celui-ci, le ballonnet (18) ayant une partie d'extrémité proximale, une partie d'extrémité distale, une partie centrale (30), et des premiers moyens lubrifiants associés audit cathéter à ballonnet, **caractérisé en ce que** lesdits premiers moyens lubrifiants sont collés sur le cathéter à ballonnet (10) et sont élaborés et positionnés de manière à ce que l'on ait une lubrification relativement plus importante pour une grande partie du tube (22) que pour au moins une partie du ballonnet (18).

2. Cathéter selon la revendication 1, dans lequel les premiers moyens lubrifiants sont élaborés et positionnés de manière à fournir un revêtement lubrifiant sur la partie (18) formant arbre, et au moins une partie du ballonnet (22) n'est recouverte d'aucun revêtement.

3. Cathéter selon la revendication 2, dans lequel la partie centrale du ballonnet (30) n'est recouverte d'aucun revêtement.

4. Cathéter selon la revendication 3, comportant en outre un revêtement lubrifiant sur la partie d'extrémité distale du ballonnet.

5. Cathéter selon la revendication 4, dans lequel la partie d'extrémité proximale du ballonnet comporte aussi un revêtement lubrifiant.

6. Cathéter selon la revendication 1, comportant des seconds moyens lubrifiants qui sont relativement moins lubrifiants que les premiers moyens lubrifiants.

7. Cathéter selon la revendication 1, dans lequel les premiers moyens lubrifiants sont élaborés et positionnés de manière à fournir un revêtement lubrifiant sur le tube (22), ledit cathéter comportant en outre des seconds moyens lubrifiants élaborés et positionnés de manière a fournir un revêtement lubrifiant sur au moins une partie du ballonnet, ledit revêtement lubrifiant positionné sur le ballonnet étant moins lubrifiant que le revêtement lubrifiant positionné sur le tube.

8. Cathéter selon la revendication 7, dans lequel la majeure partie du revêtement, qui est aussi le revêtement le plus fortement lubrifiant, est constituée d'une composition d'oxyde de polyéthylène.

9. Cathéter selon la revendication 7, dans lequel le revêtement moins lubrifiant est constitué de silicone.

10. Cathéter selon la revendication 7, dans lequel le revêtement moins lubrifiant ne recouvre qu'une partie centrale du ballonnet (30).

11. Cathéter selon l'une quelconque des revendications précédentes, comportant un ballonnet (18) ayant des extrémités opposées, un cône (32, 36) et une ceinture (34, 38) à chaque extrémité, une partie d'extrémité distale (20), des régions surfaciques et des revêtements lubrifiants associés aux régions surfaciques, des revêtements plus lubrifiant et relativement moins lubrifiant associés aux régions surfaciques étant positionnés d'une manière prédéterminée, le tube (22) comportant un revêtement relativement fortement lubrifiant collé sur une importante longueur de celui-ci en s'étendant à partir du ballonnet (18) vers l'extrémité proximale du tube (26), les deux cônes du ballonnet (32, 36) comportant un revêtement relativement fortement lubrifiant qui est similaire à celui de la partie d'extrémité distale (20), au moins une partie importante du corps de ballonnet étant enduite d'un revêtement lubrifiant qui est relativement moins lubrifiant que ledit revêtement fortement lubrifiant.

12. Cathéter selon la revendication 11, dans lequel le corps de ballonnet entier est recouvert du revêtement moins lubrifiant.

13. Cathéter selon la revendication 11, dans lequel la partie centrale du corps de ballonnet (30) n'est recouverte d'aucun revêtement.

14. Cathéter selon la revendication 11, dans lequel seule la partie centrale du corps (30) de ballonnet est recouverte du revêtement moins lubrifiant.

15. Cathéter selon la revendication 11, dans lequel la partie d'extrémité proximale du tube (26) n'est recouverte d'aucun revêtement.

16. Cathéter selon la revendication 11, dans lequel au moins la ceinture distale du ballonnet (34) est aussi recouverte du revêtement fortement lubrifiant.

17. Cathéter selon la revendication 16, comportant le même revêtement fortement lubrifiant sur la ceinture proximale du ballonnet (38).

18. Cathéter selon la revendication 11, dans lequel seul le cône distal (32) est recouvert du revêtement fortement lubrifiant.

19. Cathéter selon la revendication 18, dans lequel la partie centrale du ballonnet (30) n'est recouverte d'aucun revêtement.

20. Cathéter selon la revendication 18, dans lequel la partie centrale du ballonnet (30) est recouverte du revêtement moins lubrifiant.
